(19) Europäisches Patentamt
European Patent Office
Office européen des brevets

(11) Veröffentlichungsnummer: **0 551 821 A1**

(12) # EUROPÄISCHE PATENTANMELDUNG

(21) Anmeldenummer: **93100080.6**

(22) Anmeldetag: **05.01.93**

(51) Int. Cl.5: **C07D 261/18**, C07D 413/12, A01N 43/80

(30) Priorität: **17.01.92 DE 4201047**

(43) Veröffentlichungstag der Anmeldung: **21.07.93 Patentblatt 93/29**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Hallenbach, Werner, Dr.**
**Lichtenberger Strasse 68**
**W-4019 Monheim(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Isoxazolcarbonsäure-Derivate und ihre Verwendung als Herbizide.**

(57) Die Erfindung betrifft Isoxazolcarbonsäurederivate der allgemeinen Formel (I)

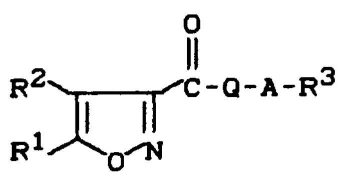

(I)

in welcher
A für Alkandiyl steht,
Q für Sauerstoff, Schwefel, Imino (NH) oder Alkylimino (N-Alkyl) steht,
$R^1$ für einen jeweils gegebenenfalls substituierten Rest der Reihe Alkyl, Cycloalkyl oder Phenyl steht,
$R^2$ für Wasserstoff, Halogen oder gegebenenfalls substituiertes Alkyl steht und
$R^3$ für jeweils gegebenenfalls substituiertes Cyclohexyl oder Phenyl steht,
ausgenommen die Verbindungen:
N-Benzyl-5-cyclopropyl-isoxazol-3-carbonsäureamid N-(2-Phenyl-ethyl)-5-methyl-isoxazol-3-carbonsäureamid und N-Benzyl-5-methyl-isoxazol-3-carbonsäureamid, ein Verfahren zu ihrer Herstellung und ihre Verwendung als Herbizide.

EP 0 551 821 A1

Die Erfindung betrifft neue Isoxazolcarbonsäurederivate, ein Verfahren zu ihrer Herstellung und ihre Verwendung als Herbizide.

Eine Reihe von Isoxazolcarbonsäurederivaten sind bereits als Herbizide oder als Zwischenprodukte für Herbizide bekannt geworden (vgl. EP-A 418667). Sie haben bisher jedoch keine Bedeutung als Herbizide erlangt.

Weitere Isoxazolcarbonsäurederivate sind als Pharmazeutika oder als Zwischenprodukte für die Herstellung von Pharmazeutika bekannt geworden (vgl. DE-OS 2950380; JP-A 45007054 - zitiert in Chem. Abstracts 72: 132705e).

Es wurden nun neue Isoxazolcarbonsäurederivate der allgemeinen Formel (I) gefunden,

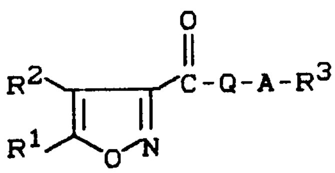

(I)

in welcher

A für Alkandiyl steht,

Q für Sauerstoff, Schwefel, Imino (NH) oder Alkylimino (N-Alkyl) steht,

$R^1$ für einen jeweils gegebenenfalls substituierten Rest der Reihe Alkyl, Cycloalkyl oder Phenyl steht,

$R^2$ für Wasserstoff, Halogen oder gegebenenfalls substituiertes Alkyl steht und

$R^3$ für jeweils gegebenenfalls substituiertes Cyclohexyl, Phenyl, Pyridyl, Furyl oder Thienyl steht,

wobei die folgenden, vorbekannten Verbindungen durch Disclaimer ausgenommen sind:

N-Benzyl-5-cyclopropyl-isoxazol-3-carbonsäureamid (vgl. EP-A 418667, S. 71), N-(2-Phenyl-ethyl)-5-methyl-isoxazol-3-carbonsäureamid (vgl. DE-OS 2950380) und N-Benzyl-5-methyl-isoxazol-3-carbonsäureamid (vgl. JP-A 45007054 - zitiert in Chem. Abstracts 72: 132705e).

Man erhält die neuen Verbindungen der Formel (I), wenn man Isoxazolcarbonsäurederivate der allgemeinen Formel (II)

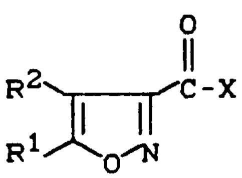

(II)

in welcher

X für Halogen oder Alkoxy steht sowie

$R^1$ und $R^2$ die oben angegebenen Bedeutungen haben,

mit nucleophilen Verbindungen der allgemeinen Formel (III)

$H\text{-}Q\text{-}A\text{-}R^3$ (III)

in welcher

A, Q und $R^3$ die oben angegebenen Bedeutungen haben,

gegebenenfalls in Gegenwart eines Säureakzeptors und gegebenenfalls in Gegenwart eines Verdünnungsmittels umsetzt.

Die neuen Isoxazolcarbonsäurederivate der allgemeinen Formel (I) zeichnen sich durch starke herbizide Wirksamkeit aus.

Gegenstand der Erfindung sind vorzugsweise Verbindungen der Formel (I), in welcher

A für $C_1$-$C_4$-Alkandiyl steht,

Q für Sauerstoff, Schwefel, Imino (NH) oder $C_1$-$C_4$-Alkylimino steht,

$R^1$ für einen jeweils gegebenenfalls durch Halogen oder $C_1$-$C_4$-Alkoxy substituierten Rest der Reihe $C_1$-$C_6$-Alkyl, $C_3$-$C_6$-Cycloalkyl steht, oder für gegebenenfalls durch Halogen oder durch $C_1$-$C_4$-Alkyl oder $C_1$-$C_4$-Alkoxy (welche jeweils gegebenenfalls durch Halogen substituiert sind) substituiertes Phenyl steht,

$R^2$ für Wasserstoff, Halogen oder gegebenenfalls durch Halogen substituiertes $C_1$-$C_{10}$-Alkyl steht und

$R^3$ für jeweils gegebenenfalls durch Nitro, Cyano, Carboxy, Halogen oder durch $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl (welche jeweils gegebenenfalls durch Halogen substituiert sind) substituiertes Cyclohexyl, Phenyl, Pyridyl, Furyl oder Thienyl steht,

wobei die Verbindungen N-Benzyl-5-cyclopropylisoxazol-3-carbonsäureamid, N-(2-Phenyl-ethyl)-5-methyl-isoxazol-3-carbonsäureamid und N-Benzyl-5-methyl-isoxazol-3-carbonsäureamid durch Disclaimer ausgenommen sind.

Die in der Definition der erfindungsgemäßen Verbindungen genannten Kohlenwasserstoffgruppen, wie Alkyl oder Alkandiyl, auch in Verbindungen mit Heteroatomen, wie in Alkoxy, sind jeweils geradkettig oder verzweigt.

Die Erfindung betrifft insbesondere Verbindungen der Formel (I), in welcher

A für Methylen ($-CH_2-$), Ethan-1,2-diyl (Dimethylen, $-CH_2CH_2-$),

Ethan-1,1-diyl 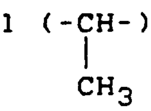, Propan-1,1-diyl

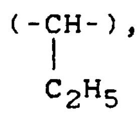, Propan-1,2-diyl 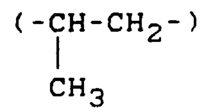

oder Propan-1,3-diyl ($-CH_2CH_2CH_2-$) steht,

Q für Sauerstoff, Imino (NH) oder Methylimino ($NCH_3$) steht,

$R^1$ für einen jeweils gegebenenfalls durch Fluor, Chlor, Methoxy oder Ethoxy substituierten Rest der Reihe Methyl, Ethyl, Propyl, Isopropyl, Butyl, Isobutyl, sec-Butyl oder tert-Butyl, oder für jeweils gegebenenfalls durch Fluor, Chlor, Methyl oder Ethyl substituiertes Cyclopropyl, Cyclobutyl, Cyclopentyl oder Cyclohexyl steht, oder für Phenyl, welches gegebenenfalls durch Fluor, Chlor, Methyl oder Methoxy substituiert ist, steht,

$R^2$ für Wasserstoff, Chlor oder Methyl steht und

$R^3$ für jeweils gegebenenfalls durch Nitro, Cyano, Carboxy, Fluor, Chlor, Methyl, Ethyl, Trifluormethyl, Methoxy, Ethoxy, Difluormethoxy oder Trifluormethoxy substituiertes Cyclohexyl, Phenyl, Pyridyl, Furyl oder Thienyl steht,

mit Ausnahme der durch Disclaimer ausgenommenen Verbindungen.

Verwendet man beispielsweise 5-Methyl-isoxazol-3-carbonsäurechlorid und 1-Phenyl-ethylamin als Ausgangsstoffe, so kann der Reaktionsablauf beim erfindungsgemäßen Verfahren durch das folgende Formelschema skizziert werden:

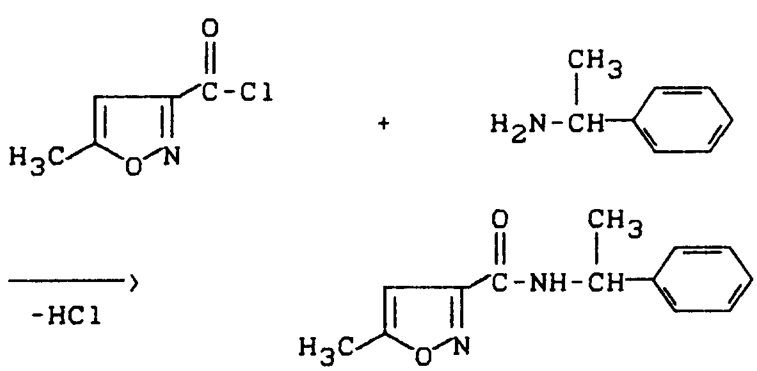


Die beim erfindungsgemäßen Verfahren zur Herstellung von Verbindungen der Formel (I) als Ausgangsstoffe zu verwendenden Isoxazolcarbonsäurederivate sind durch die Formel (II) allgemein definiert.

In Formel (II) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I)

vorzugsweise bzw. als insbesondere bevorzugt für $R^1$ und $R^2$ angegeben wurden;

X steht vorzugsweise für Chlor, Methoxy oder Ethoxy.

Die Ausgangsstoffe der Formel (II) sind bekannt und/oder können nach an sich bekannten Verfahren hergestellt werden (vgl. JP-A 49000187 - zitiert in Chem. Abstracts 81: 49671f; DE-P 1670952; US-P 4189581; DE-OS 2950380; J. Heterocycl. Chem. 19 (1982), 557-560; Tetrahedron 43 (1987) 235-242; EP-A 418667; Herstellungsbeispiele).

Die beim erfindungsgemäßen Verfahren zur Herstellung von Verbindungen der Formel (I) weiter als Ausgangsstoffe zu verwendenden nucleophilen Verbindungen sind durch die Formel (III) allgemein definiert.

In Formel (III) haben A, Q und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q und $R^3$ angegeben wurden.

Die Ausgangsstoffe der Formel (III) sind bekannte organische Synthesechemikalien.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und - hydrogencarbonate, wie Natrium- und Kalium-carbonat oder - hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tertbutylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflachen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Ouizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

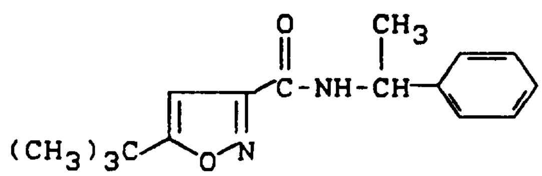

Eine Lösung von 6,0 g (32 mMol) 5-tert-Butyl-isoxazol-3-carbonsäurechlorid in 10 ml Chloroform wird bei 20 °C unter Rühren zu einer Mischung aus 3,9 g (32 mMol) 1-Phenyl-ethylamin, 5,5 ml (39,5 mMol) Triethylamin und 40 ml Chloroform tropfenweise gegeben. Das Reaktionsgemisch wird 15 Stunden bei 20 °C gerührt, dann auf etwa das gleiche Volumen Wasser gegossen und durchgeschüttelt. Die organische Phase wird abgetrennt, mit 2N-Salzsäure und dann mit gesättigter Natriumhydrogencarbonat-Lösung gewaschen, mit Natriumsulfat getrocknet und filtriert. Vom Filtrat wird das Lösungsmittel im Wasserstrahlvakuum sorgfältig abdestilliert. Nach Entgasen im Ölpumpenvakuum verbleibt das Reaktionsprodukt als fester Rückstand.

Man erhält 7,9 g (91% der Theorie) (R/S)-N-(1-Phenylethyl)-5-tert-butyl-isoxazol-3-carbonsäureamid vom Schmelzpunkt 88 °C.

Analog Beispiel 1 und entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

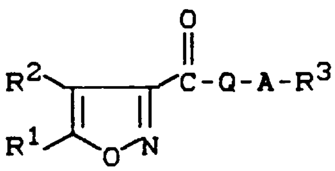

(I)

Tabelle 1: Beispiele für die Verbindungen der Formel (I)

| Bsp.-Nr. | A | Q | $R^1$ | $R^2$ | $R^3$ | Schmelzpunkt (°C) |
|---|---|---|---|---|---|---|
| 2 | (R/S)-CH-<br>$CH_3$ | NH | $C(CH_3)_3$ | H | 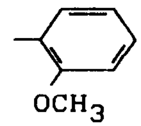 | 134 |
| 3 | (S)-CH-<br>$CH_3$ | NH | $C(CH_3)_3$ | H | 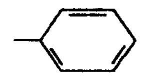 | 92 |
| 4 | (R/S)-$CHCH_2CH_2$-<br>$CH_3$ | NH | $C(CH_3)_3$ | H | 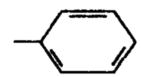 | 70 |
| 5 | (R)-CH-<br>$CH_3$ | NH | $C(CH_3)_3$ | H | 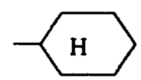 | 93 |
| 6 | (R/S)-CH-<br>$CH_3$ | NH | $C(CH_3)_3$ | H | 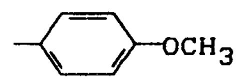 | 92 |
| 7 | -$CH_2CH_2$- | NH | $C(CH_3)_3$ | H | 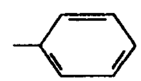 | 93 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 8 | (R)-CH-<br>$CH_3$ | NH | $C(CH_3)_3$ | H | 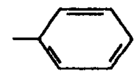 | 91 |
| 9 | $-CH_2-$ | O | $C(CH_3)_3$ | H | 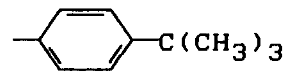 | 53 |
| 10 | $-CH_2CH_2-$ | O | $C(CH_3)_3$ | H | 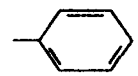 | (Öl) |
| 11 | (R/S)-CH-<br>$CH_3$ | NH | $C(CH_3)_3$ | H | 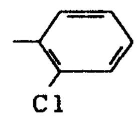 | 128 |
| 12 | (R/S)-CH-<br>$CH_3$ | NH | $C(CH_3)_3$ | H | 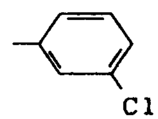 | 114 |
| 13 | (R/S)-CH-<br>$CH_3$ | NH | $C(CH_3)_3$ | H | 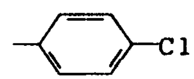 | 137 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 14 | $-CH_2-$ | NH | $C(CH_3)_3$ | H | 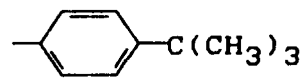 | 97 |
| 15 | (R/S)-CH-<br>\|<br>$CH_3$ | NH | $C(CH_3)_3$ | H | 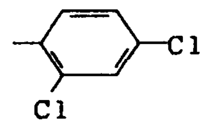 | 126 |
| 16 | (R/S)-CH-<br>\|<br>$CH_3$ | NH | $C(CH_3)_3$ | H | 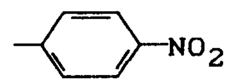 | 107 |
| 17 | (R/S)-CH-<br>\|<br>$CH_3$ | NH | $C(CH_3)_3$ | H | 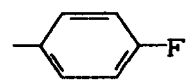 | 135 |
| 18 | (S)-CH-<br>\|<br>$CH_3$ | NH | $C(CH_3)_3$ | H | 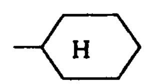 | 97 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 19 | $-CH_2-$ | NH | $C(CH_3)_3$ | H | 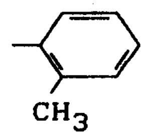 | 74 |
| 20 | $-CH_2-$ | NH | $C(CH_3)_3$ | H | 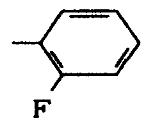 | 107 |
| 21 | $-CH_2-$ | NH | $C(CH_3)_3$ | H | 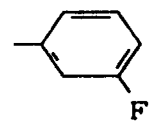 | 81 |
| 22 | $-CH_2-$ | NH | $C(CH_3)_3$ | H | 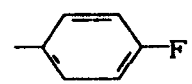 | 87 |
| 23 | $-CH_2-$ | NH | $C(CH_3)_3$ | H | 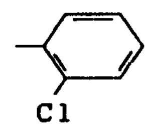 | 107 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 24 | $-CH_2-$ | NH | $C(CH_3)_3$ | H | 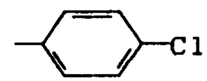 | 127 |
| 25 | $-CH_2-$ | NH | $C(CH_3)_3$ | H | 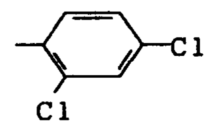 | 115 |
| 26 | $-CH_2-$ | NH | $C(CH_3)_3$ | H | 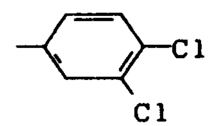 | 114 |
| 27 | $-CH_2-$ | NH | $C(CH_3)_3$ | H | 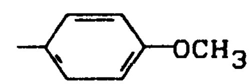 | (amorph) |
| 28 | $-CH_2-$ | NH | $C(CH_3)_3$ | H | 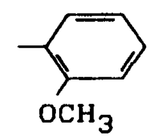 | (amorph) |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 29 | $-CH_2-$ | NH | $C(CH_3)_3$ | H | 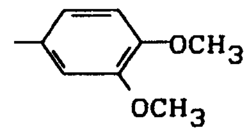 | 94 |
| 30 | $-CH_2-$ | NH | $C(CH_3)_3$ | H | 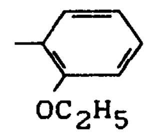 | 64 |
| 31 | (R/S)-$CH(CH_3)$- | NH | 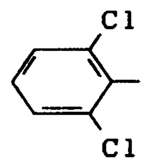 | H | 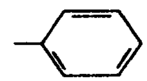 | (amorph) |
| 32 | (R/S)-$CH(CH_3)$- | NH | 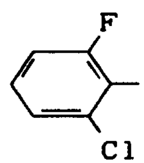 | H | 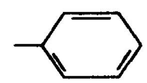 | 124 |
| 33 | $-CH_2-$ | NH | 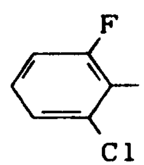 | H | 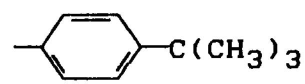 | 115 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 34 | (R/S)-CH(-$CH_3$)- | $NCH_3$ | 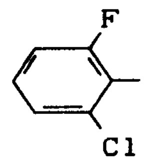 | H | 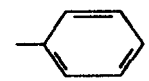 | (amorph) |
| 35 | (R/S)-CH(-$CH_3$)- | $NCH_3$ | 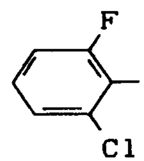 | H | 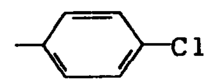 | (amorph) |
| 36 | $-CH_2-$ | O | 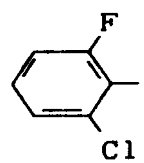 | H | 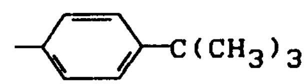 | 56 |
| 37 | $-CH_2-$ | NH | $C(CH_3)_3$ | H | 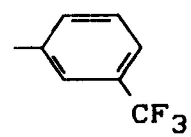 | 83 |
| 38 | $-CH_2-$ | NH | $C(CH_3)_3$ | H | 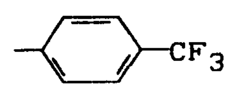 | 119 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 39 | $-CH_2-$ | NH | $C(CH_3)_3$ | H | 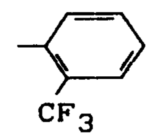 | 89 |
| 40 | $-CH_2-$ | NH | $C(CH_3)_3$ | H | 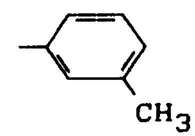 | 82 |
| 41 | $-CH_2-$ | NH | $C(CH_3)_3$ | H | 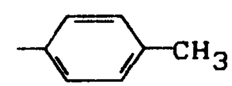 | 111 |
| 42 | $-CH_2-$ | NH | $C(CH_3)_3$ | H | 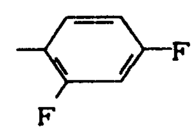 | 99 |
| 43 | $-CH_2-$ | NH | $C(CH_3)_3$ | H | 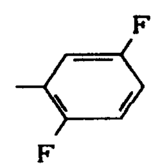 | 110 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 44 | $-CH_2-$ | NH | $C(CH_3)_3$ | H | 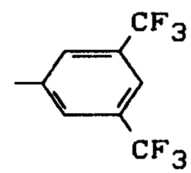 | 118 |
| 45 | $-CH_2-$ | NH | $C(CH_3)_3$ | H | 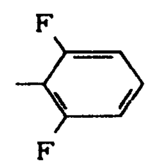 | 97 |
| 46 | $-CH_2-$ | NH | $C(CH_3)_3$ | H | 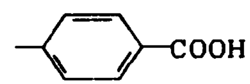 | 187 |
| 47 | $-CH_2-$ | NH | $C(CH_3)_3$ | H | 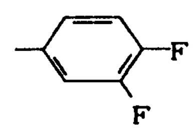 | 79 |
| 48 | $-CH_2-$ | NH | $C(CH_3)_3$ | H | 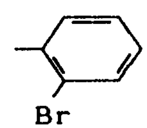 | 96 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 49 | $-CH_2-$ | NH | $C(CH_3)_3$ | H | 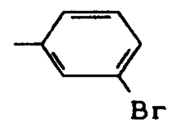 | 62 |
| 50 | $-CH_2-$ | NH | $C(CH_3)_3$ | H | 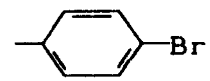 | 136 |
| 51 | $-CH_2-$ | NH | $C(CH_3)_3$ | H | 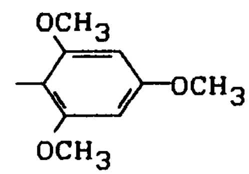 | 121 |
| 52 | $-CH_2-$ | NH | $C(CH_3)_3$ | H | 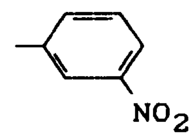 | 94 |
| 53 | $-CH_2-$ | NH | $C(CH_3)_3$ | H | 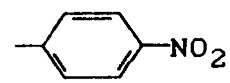 | 120 |
| 54 | $(R/S)-CH(CH_3)-$ | NH | $CH_3$ | H | 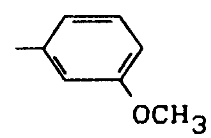 | |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 55 | (R/S)-CH-<br>$CH_3$ | NH | $CH_2CH(CH_3)_2$ | H | 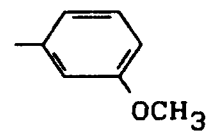 | |
| 56 | (R/S)-CH-<br>$CH_3$ | NH | $CH(CH_3)_2$ | H | 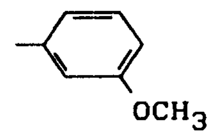 | |
| 57 | (R/S)-CH-<br>$CH_3$ | NH | $CH_3$ | H | 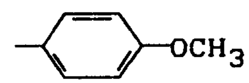 | |
| 58 | (R/S)-CH-<br>$CH_3$ | NH | $CH_2CH(CH_3)_2$ | H | 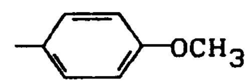 | |
| 59 | (R/S)-CH-<br>$CH_3$ | NH | $CH(CH_3)_2$ | H | 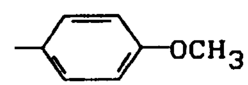 | |

**Tabelle 1** - Fortsetzung

| Bsp.-Nr. | A | Q | $R^1$ | $R^2$ | $R^3$ | Schmelzpunkt (°C) |
|---|---|---|---|---|---|---|
| 60 | (R/S)-CH-<br>$CH_3$ | NH | $CH_3$ | H | 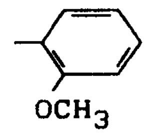 | |
| 61 | (R/S)-CH-<br>$CH_3$ | NH | $CH_2CH(CH_3)_2$ | H | 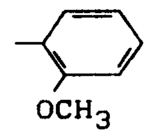 | |
| 62 | (R/S)-CH-<br>$CH_3$ | NH | $CH(CH_3)_2$ | H | 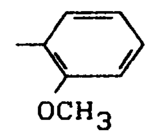 | |
| 63 | (R/S)-CH-<br>$CH_3$ | NH | $CH_3$ | H | 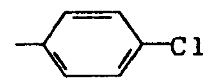 | |
| 64 | (R/S)-CH-<br>$CH_3$ | NH | $CH_2CH(CH_3)_2$ | H | 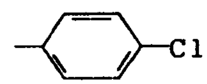 | |
| 65 | (R/S)-CH-<br>$CH_3$ | NH | $CH(CH_3)_2$ | H | 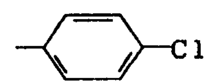 | |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 66 | (R/S)-CH-$CH_2$-$CH_2$-<br>\|<br>$CH_3$ | NH | $CH(CH_3)_2$ | H | 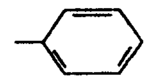 | |
| 67 | (R/S)-CH-<br>\|<br>$CH_3$ | NH | $CH_2CH(CH_3)_2$ | H | 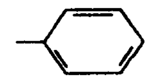 | |
| 68 | (R/S)-CHCH$_2$CH$_2$-<br>\|<br>$CH_3$ | NH | $CH(CH_3)_2$ | H | 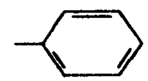 | |
| 69 | (R/S)-CH-<br>\|<br>$CH_3$ | NH | $CH(CH_3)_2$ | H | 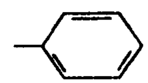 | |
| 70 | (R/S)-CH-<br>\|<br>$CH_3$ | NH | $CH_3$ | H | 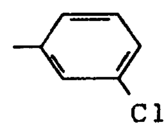 | |
| 71 | (R/S)-CH-<br>\|<br>$CH_3$ | NH | $CH_2CH(CH_3)_2$ | H | 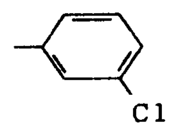 | |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 72 | (R/S)-CH-<br>$CH_3$ | NH | $CH(CH_3)_2$ | H | 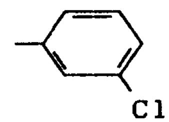 | |
| 73 | (R/S)-CH-<br>$CH_3$ | NH | $CH_3$ | H | 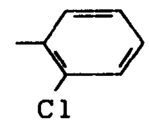 | |
| 74 | (R/S)-CH-<br>$CH_3$ | NH | $CH_2CH(CH_3)_2$ | H | 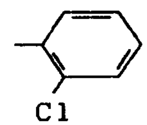 | |
| 75 | (R/S)-CH-<br>$CH_3$ | NH | $CH(CH_3)_2$ | H | 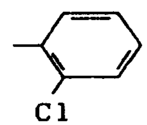 | |
| 76 | (R/S)-CH-<br>$CH_3$ | NH | $C(CH_3)_3$ | H | 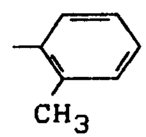 | |
| 77 | (R/S)-CH-<br>$CH_3$ | NH | $C(CH_3)_3$ | H | 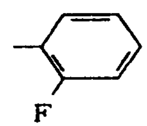 | |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 78 | 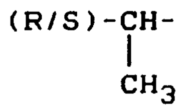 | NH | $CH(CH_3)_2$ | H | 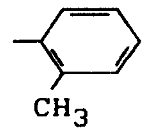 | |
| 79 | 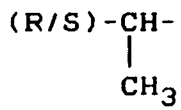 | NH | $CH_2CH(CH_3)_2$ | H | 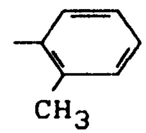 | |
| 80 | 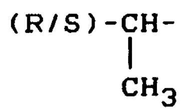 | NH | $CH_2CH(CH_3)_2$ | H | 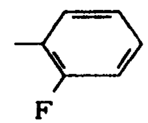 | |
| 81 | $-CH_2-$ | NH | $CH_2CH(CH_3)_2$ | H | 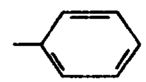 | |
| 82 | 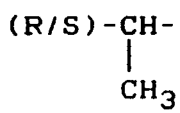 | NH | $CH_3$ | H | 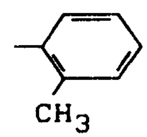 | |
| 83 | 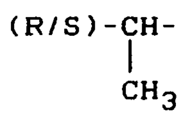 | NH | $CH_3$ | H | 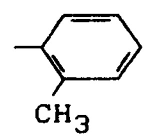 | |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 84 | $-CH_2-$ | NH | $CH_3$ | H | 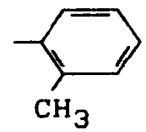 | 84 |
| 85 | $-CH_2-$ | NH | $CH_3$ | H | 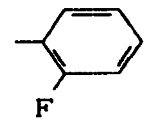 | 102 |
| 86 | $-CH_2-$ | NH | $CH_3$ | H | 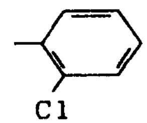 | 95 |
| 87 | $-CH_2-$ | NH | $CH_3$ | H | 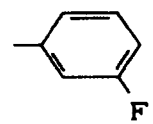 | 79 |
| 88 | $-CH_2-$ | NH | $CH_3$ | H | 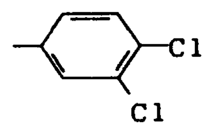 | 136 |
| 89 | $-CH_2-$ | NH | $CH_3$ | H | 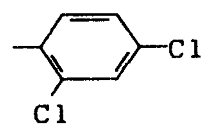 | 143 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 90 | $-CH_2-$ | NH | $CH_3$ | H | 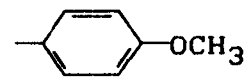 | 89 |
| 91 | $-CH_2-$ | NH | $CH_3$ | H | 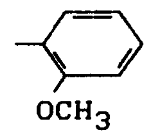 | |
| 92 | $-CH_2-$ | NH | $CH_3$ | H | 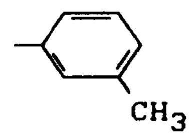 | 64 |
| 93 | $-CH_2-$ | NH | $CH_3$ | H | 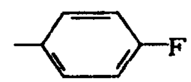 | 118 |
| 94 | $-CH_2-$ | NH | $CH_3$ | H | 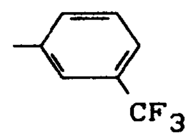 | 81 |
| 95 | $-CH_2-$ | NH | $CH_3$ | H | 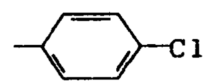 | 143 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 96 | $-CH_2-$ | NH | $CH_3$ | H | 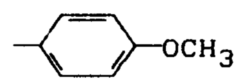 | 109 |
| 97 | $-CH_2-$ | NH | $CH_3$ | H | 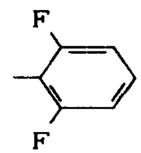 | 125 |
| 98 | $-CH_2-$ | NH | $CH_3$ | H | 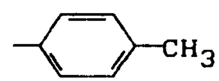 | 89 |
| 99 | $-CH_2-$ | NH | $CH_3$ | H | 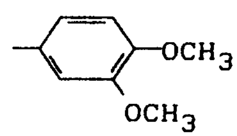 | 115 |
| 100 | $-CH_2-$ | NH | $CH_3$ | H | 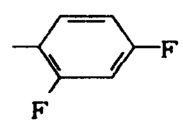 | 128 |
| 101 | $-CH_2-$ | NH | $CH_3$ | H | 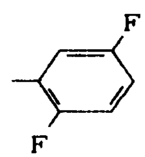 | 106 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 102 | $-CH_2-$ | NH | $CH_3$ | H | 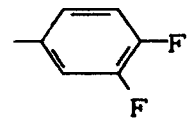 | 115 |
| 103 | $-CH_2-$ | NH | $CH_3$ | H | 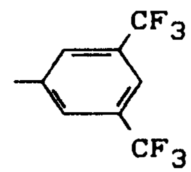 | 101 |
| 104 | $-CH_2-$ | NH | $CH_3$ | H | 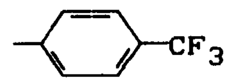 | 146 |
| 105 | $-CH_2-$ | NH | $CH_3$ | H | 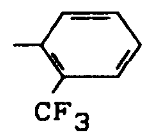 | 113 |
| 106 | $-CH_2-$ | NH | $CH_3$ | H | 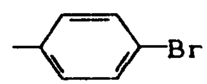 | 137 |
| 107 | $-CH_2-$ | NH | $CH(CH_3)_2$ | H | 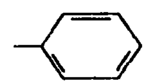 | |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 108 | $-CH_2-$ | NH | $CH(CH_3)_2$ | H | 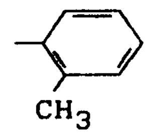 | |
| 109 | $-CH_2-$ | NH | $CH_2CH(CH_3)_2$ | H | 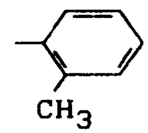 | |
| 110 | $-CH_2-$ | NH | $CH_2CH(CH_3)_2$ | H | 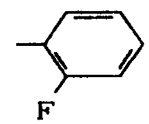 | |
| 111 | $-CH_2-$ | NH | $CH_2CH(CH_3)_2$ | H | 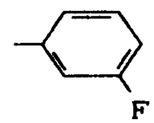 | |
| 112 | $-CH_2-$ | NH | $CH_2CH(CH_3)_2$ | H | 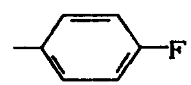 | |
| 113 | $-CH_2-$ | NH | $CH(CH_3)_2$ | H | 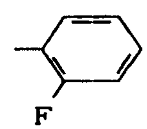 | |

**Tabelle 1** - Fortsetzung

| Bsp.-Nr. | A | Q | $R^1$ | $R^2$ | $R^3$ | Schmelzpunkt (°C) |
|---|---|---|---|---|---|---|
| 114 | $-CH_2-$ | NH | $CH(CH_3)_2$ | H | 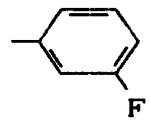 | |
| 115 | $-CH_2-$ | NH | $CH(CH_3)_2$ | H | 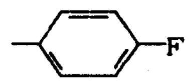 | |
| 116 | $-CH_2-$ | NH | $C(CH_3)_3$ | H | 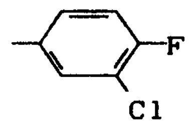 | 113 |
| 117 | $-CH_2-$ | NH | $C(CH_3)_3$ | H | 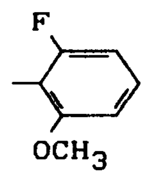 | 67 |
| 118 | $-CH_2-$ | NH | $C(CH_3)_3$ | H | 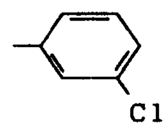 | 54 |
| 119 | $-CH_2-$ | NH | $C(CH_3)_3$ | H | 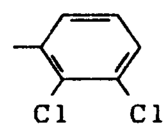 | 129 |

Tabelle 1 - Fortsetzung

| Bsp.-Nr. | A | Q | $R^1$ | $R^2$ | $R^3$ | Schmelzpunkt (°C) |
|---|---|---|---|---|---|---|
| 120 | $-CH_2-$ | NH | $C(CH_3)_3$ | H | 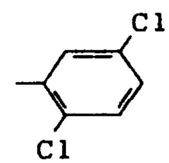 | 127 |
| 121 | $-CH_2-$ | NH | $C(CH_3)_3$ | H | 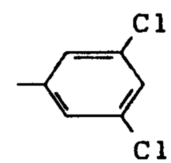 | 131 |
| 122 | $-CH_2-$ | NH | $C(CH_3)_3$ | H | 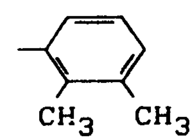 | 60 |
| 123 | $-CH_2-$ | NH | $C(CH_3)_3$ | H | 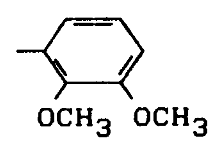 | 77 |
| 124 | $-CH_2-$ | NH | $C(CH_3)_3$ | H | 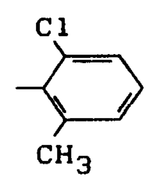 | 119 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 125 | $-CH_2-$ | NH | $C(CH_3)_3$ | H | 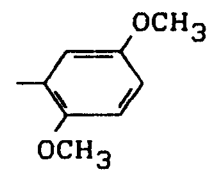 | 84 |
| 126 | $-CH_2-$ | NH | $C(CH_3)_3$ | H | 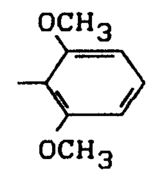 | 116 |
| 127 | $-CH_2-$ | NH | $C(CH_3)_3$ | H | 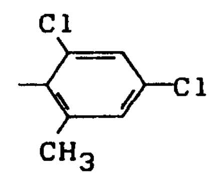 | 109 |
| 128 | $-CH_2-$ | NH | $C(CH_3)_3$ | H | 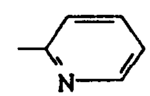 | |
| 129 | $-CH_2-$ | NH | $C(CH_3)_3$ | H | 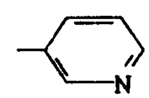 | 89 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 130 | $-CH_2-$ | NH | $C(CH_3)_3$ | H | 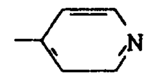 | 64 |
| 131 | (R/S)-CH-<br>$CH_3$ | NH | $C(CH_3)_3$ | H | 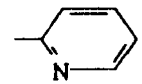 | |
| 132 | $-CH_2-$ | NH | $C(CH_3)_3$ | H | 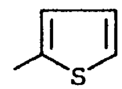 | 81 |
| 133 | (R/S)-CH-<br>$C_2H_5$ | NH | $C(CH_3)_3$ | H | 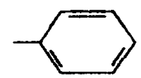 | 106 |
| 134 | $-CH_2-$ | NH | $C(CH_3)_3$ | H | 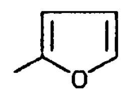 | 59 |
| 135 | (R/S)-CH-<br>$CH_3$ | NH | $C(CH_3)_3$ | H | 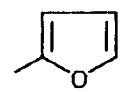 | |
| 136 | (R/S)-CH-<br>$CH_3$ | NH | $C(CH_3)_3$ | H | 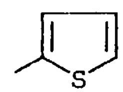 | 50 |

<u>Tabelle 1</u> - Fortsetzung

| Bsp.-Nr. | A | Q | $R^1$ | $R^2$ | $R^3$ | Schmelzpunkt (°C) |
|---|---|---|---|---|---|---|
| 137 | $-CH_2-$ | NH | $C(CH_3)_3$ | H | 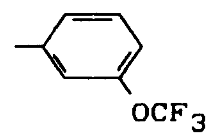 | 67 |
| 138 | $-CH_2-$ | NH | $C(CH_3)_3$ | H | 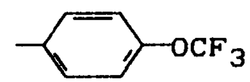 | 103 |
| 139 | $-CH_2-$ | NH | $C(CH_3)_3$ | H | 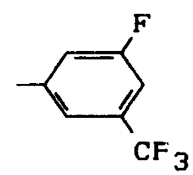 | 82 |

<u>Ausgangsstoffe der Formel (II)</u>

<u>Beispiel (II-1)</u>

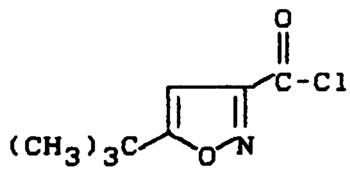

Eine Mischung aus 28,7 g (0,17 Mol) 5-tert-Butyl-isoxazol-3-carbonsäure und 70 ml Thionylchlorid wird 3 Stunden unter Rückfluß erhitzt und dann durch Destillation aufgearbeitet.

Man erhält 19 g (60% der Theorie) 5-tert-Butyl-isoxazol-3-carbonsäurechlorid vom Siedepunkt 60 °C (bei 0,5 mbar).

Anwendungsbeispiele:

Beispiel A Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werdend Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 1 und 3 bei guter Verträglichkeit gegenüber Kulturpflanzen, wie z.B. Weizen, starke Wirkung gegen Unkräuter.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Losungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt beispielsweise die Verbindung gemäß Herstellungsbeispiel 1 bei guter Verträglichkeit gegenüber Kulturpflanzen, wie z.B. Mais und Baumwolle, starke Wirkung gegen Unkräuter.

**Patentansprüche**

1. Isoxazolcarbonsäurederivate der allgemeinen Formel (I)

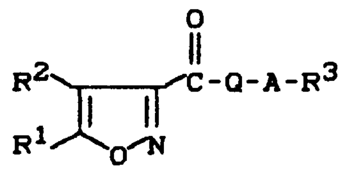

(I)

in welcher

- A für Alkandiyl steht,
- Q für Sauerstoff, Schwefel, Imino (NH) oder Alkylimino (N-Alkyl) steht,
- $R^1$ für einen jeweils gegebenenfalls substituierten Rest der Reihe Alkyl, Cycloalkyl oder Phenyl steht,
- $R^2$ für Wasserstoff, Halogen oder gegebenenfalls substituiertes Alkyl steht und
- $R^3$ für jeweils gegebenenfalls substituiertes Cyclohexyl, Phenyl, Pyridyl, Furyl oder Thienyl steht,

ausgenommen die Verbindungen:
N-Benzyl-5-cyclopropyl-isoxazol-3-carbonsäureamid N-(2-Phenyl-ethyl)-5-methyl-isoxazol-3-carbonsäureamid und N-Benzyl-5-methyl-isoxazol-3-carbonsäureamid.

**2.** Isoxazolcarbonsäurederivate der allgemeinen Formel (I) gemäß Anspruch 1, dadurch gekennzeichnet, daß

- A für $C_1$-$C_4$-Alkandiyl steht,
- Q für Sauerstoff, Schwefel, Imino (NH) oder $C_1$-$C_4$-Alkylimino steht,
- $R^1$ für einen jeweils gegebenenfalls durch Halogen oder $C_1$-$C_4$-Alkoxy substituierten Rest der Reihe $C_1$-$C_6$-Alkyl, $C_3$-$C_6$-Cycloalkyl steht, oder für gegebenenfalls durch Halogen oder durch $C_1$-$C_4$-Alkyl oder $C_1$-$C_4$-Alkoxy (welche jeweils gegebenenfalls durch Halogen substituiert sind) substituiertes Phenyl steht,
- $R^2$ für Wasserstoff, Halogen oder gegebenenfalls durch Halogen substituiertes $C_1$-$C_{10}$-Alkyl steht und
- $R^3$ für jeweils gegebenenfalls durch Nitro, Cyano, Carboxy, Halogen oder durch $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl (welche jeweils gegebenenfalls durch Halogen substituiert sind) substituiertes Cyclohexyl, Phenyl, Pyridyl, Furyl oder Thienyl steht,

ausgenommen die Verbindungen N-Benzyl-5-cyclopropylisoxazol-3-carbonsäureamid, N-(2-Phenylethyl)-5-methyl-isoxazol-3-carbonsäureamid und N-Benzyl-5-methyl-isoxazol-3-carbonsäureamid.

**3.** Isoxazolcarbonsäurederivate der allgemeinen Formel (I) gemäß Anspruch 1, dadurch gekennzeichnet, daß

- A für Methylen ($-CH_2-$), Ethan-1,2-diyl (Dimethylen, $-CH_2CH_2-$),

Ethan-1,1-diyl 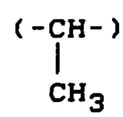, Propan-1,1-diyl

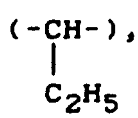, Propan-1,2-diyl 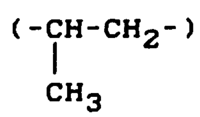

oder Propan-1,3-diyl ($-CH_2CH_2CH_2-$) steht,

- Q für Sauerstoff, Imino (NH) oder Methylimino ($NCH_3$) steht,
- $R^1$ für einen jeweils gegebenenfalls durch Fluor, Chlor, Methoxy oder Ethoxy substituierten Rest der Reihe Methyl, Ethyl, Propyl, Isopropyl, Butyl, Isobutyl, sec-Butyl oder tert-Butyl, oder für jeweils gegebenenfalls durch Fluor, Chlor, Methyl oder Ethyl substituiertes Cyclopropyl, Cyclobutyl, Cyclopentyl oder Cyclohexyl steht, oder für Phenyl, welches gegebenenfalls durch Fluor, Chlor, Methyl oder Methoxy substituiert ist, steht,
- $R^2$ für Wasserstoff, Chlor oder Methyl steht und
- $R^3$ für jeweils gegebenenfalls durch Nitro, Cyano, Carboxy, Fluor, Chlor, Methyl, Ethyl, Trifluormethyl, Methoxy, Ethoxy, Difluormethoxy oder Trifluormethoxy substituiertes Cyclohexyl, Phenyl, Pyridyl, Furyl oder Thienyl steht,

ausgenommen die Verbindungen N-Benzyl-5-cyclopropylisoxazol-3-carbonsäureamid, N-(2-Phenylethyl)-5-methyl-isoxazol-3-carbonsäureamid und N-Benzyl-5-methyl-isoxazol-3-carbonsäureamid.

**4.** Verfahren zur Herstellung von Isoxazolcarbonsäurederivaten der allgemeinen Formel (I)

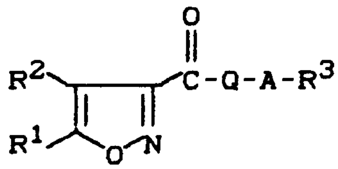

(I)

in welcher

A für Alkandiyl steht,

Q für Sauerstoff, Schwefel, Imino (NH) oder Alkylimino (N-Alkyl) steht,

$R^1$ für einen jeweils gegebenenfalls substituierten Rest der Reihe Alkyl, Cycloalkyl oder Phenyl steht,

$R^2$ für Wasserstoff, Halogen oder gegebenenfalls substituiertes Alkyl steht und

$R^3$ für jeweils gegebenenfalls substituiertes Cyclohexyl, Phenyl, Pyridyl, Furyl oder Thienyl steht,

ausgenommen die Verbindungen:

N-Benzyl-5-cyclopropyl-isoxazol-3-carbonsäureamid N-(2-Phenyl-ethyl)-5-methyl-isoxazol-3-carbonsäureamid und N-Benzyl-5-methyl-isoxazol-3-carbonsäureamid,

dadurch gekennzeichnet, daß man Isoxazolcarbonsäurederivate der allgemeinen Formel (II)

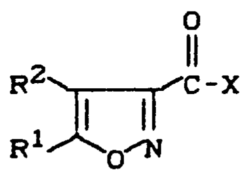

(II)

in welcher

X für Halogen oder Alkoxy steht sowie

$R^1$ und $R^2$ die oben angegebenen Bedeutungen haben,

mit nucleophilen Verbindungen der allgemeinen Formel (III)

$H\text{-}Q\text{-}A\text{-}R^3$ (III)

in welcher

A, Q und $R^3$ die oben angegebenen Bedeutungen haben,

gegebenenfalls in Gegenwart eines Säureakzeptors und gegebenenfalls in Gegenwart eines Verdünnungsmittels umsetzt.

**5.** Herbizide Mittel, gekennzeichnet durch einen Gehalt an mindestens einem Isoxazolcarbonsäurederivat der Formel (I) gemäß dem Anspruch 1.

**6.** Verfahren zur Bekämpfung von unerwünschten Pflanzen, dadurch gekennzeichnet, daß man Isoxazolcarbonsäurederivate der Formel (I) gemäß dem Anspruch 1 auf unerwünschte Pflanzen und/oder ihren Lebensraum einwirken läßt.

**7.** Verwendung von Isoxazolcarbonsäurederivaten der Formel (I) gemäß dem Anspruch 1 zur Bekämpfung von unerwünschten Pflanzen.

**8.** Verfahren zur Herstellung von herbiziden Mitteln, dadurch gekennzeichnet, daß man Isoxazolcarbonsäurederivate der Formel (I) gemäß dem Anspruch 1 mit Streckmitteln und/oder oberflächenaktiven

Substanzen vermischt.

Europäisches Patentamt

# EUROPÄISCHER RECHERCHENBERICHT

Nummer der Anmeldung

EP 93 10 0080

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
| --- | --- | --- | --- |
| D,X | EP-A-0 418 667 (BASF AKTIENGESELLSCHAFT) * Seite 70 - Seite 71; Ansprüche 1,4,5,7,14-152 * | 1-8 | C07D261/18<br>C07D413/12<br>A01N43/80 |
| X | DE-A-2 062 373 (FERLUX S.A.) *Seite 27;Verbindungen19,20,21;Seite28,Verbindung25* * Anspruch 1 * | 1-3 | |
| D,X | FR-A-2 459 237 (A.WASSERMANN SPA) * Anspruch 6 * | 1-3 | |
| X | CHEMICAL ABSTRACTS, vol. 72, no. 25, 22. Juni 1970, Columbus, Ohio, US; abstract no. 132705e, Seite 365 ; * Zusammenfassung * | 1-3 | |
| D | & JP-A-7 007 054 (FUJISAWA PHARMACEUTICAL CO.,LTD) 11. März 1970 | | |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
| --- | --- | --- |
| DEN HAAG | 11 MAERZ 1993 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)